# EUROPEAN PATENT SPECIFICATION

(11) **EP 2 268 206 B1**
(45) Date of publication and mention of the grant of the patent: **24.12.2014**
(21) Application number: 09719628.1
(22) Date of filing: 26.02.2009
(51) Int. Cl.: A61B 8/00, A61B 8/12, A61M 37/00, A61B 8/06, A61M 5/00

(54) **IMAGING CATHETER WITH INTEGRATED CONTRAST AGENT INJECTOR**
BILDGEBUNGSKATHETER MIT INTEGRIERTEM INJEKTIONSGERÄT FÜR KONTRASTMITTEL
CATHÉTER D'IMAGERIE AVEC INJECTEUR D'AGENT DE CONTRASTE INTÉGRÉ

(30) Priority: 13.03.2008 US 48134
(43) Date of publication of application: 05.01.2011
(73) Proprietor: Boston Scientific Scimed, Inc., Maple Grove, MN 55311-1566 (US)
(72) Inventor: KNIGHT, Jon, M., Pleasanton CA 94588 (US); TEO, Tat-Jin, Sunnyvale CA 94087 (US); ROMLEY, Richard, Tracy CA 95376 (US); LI, Wenguang, Campbell CA 95008 (US)
(74) Representative: Vossius & Partner
(86) International application number: PCT/US2009/035254
(87) International publication number: WO 2009/114278

(56) References cited:
- WO-A1-2005/070299
- GB-A- 2 417 080

## Description

### FIELD OF THE INVENTION

The present invention relates to imaging catheters, and more particularly to imaging catheters with integrated contrast agent injectors.

### BACKGROUND INFORMATION

Intravascular ultrasound (IVUS) is an established tool for obtaining ultrasound images within the vascular system. IVUS is a method by which a catheter-based transducer is used to obtain images of the lumen and vascular wall of larger vessels. This method has been described, e.g., in document GB 2 417 080 A. It is an established interventional cardiology tool for gaining insight into the size, structure, and composition of atherosclerotic plaque located within coronary arteries.

Ultrasound contrast imaging employs a contrast agent to enhance the detection and imaging of organs, blood vessels, or tissues within the body. Contrast agents may be comprised of echogenic microbubbles or other substances having ultrasound scattering properties that are distinct from those of the surrounding media (tissue and blood), thereby enhancing the visibility of organs, blood vessels or tissues containing the contrast agent. Materials having suitable sound reflection properties to be used as ultrasound contrast agents include solid particles and air or gas-filled microbubbles. Contrast agents are typically composed of particles of a size that allows them to flow freely through, but remain within, the vessels of the microcirculatory system. By increasing the visibility of organs, blood vessels or tissues being studied, contrast agents can aid the clinician in determining the presence and extent of disease or injury. Contrast imaging enables the viewing and differentiation of fine structures that are not normally distinguishable from surrounding tissue.

Contrast agent microbubbles can also be used to deliver a drug or other pharmaceutical agent in the patient. In drug delivery applications, the microbubbles encapsulate a drug or pharmaceutical agent instead of an inert gas. The microbubbles may be administered intravenously and travel through the vascular system. The drug remains encapsulated and inert, and therefore biologically unavailable, until a source of ultrasound energy bursts the microbubbles to release the drug.

Contrast agents are used primarily with non-invasive or intracardiac ultrasound imaging methods. In these methods, a contrast agent is injected into the bloodstream, e.g., by an intravenous drip or syringe. The injected contrast agent flows throughout the vascular system, not just the region of interest in the body. As a result, a larger quantity of contrast agent must be injected into the patient than needed to image the region of interest. Another problem with these methods is the difficulty of synchronizing the injection of contrast agent with imaging. This is due in part to the difficulty of estimating the time its takes the injected contrast agent to reach the region of interest, which can be as long as two minutes. In addition, these methods use two separate instruments for the injection of the contrast agent and the imaging.

Currently, contrast agents are administered by intravenous drip, or by manual injection through a syringe or guide catheter. GB 2 417 080 A, forming the closest prior art document for the invention, mentions an automatic injection of the contrast agent and an injection of the contrast agent which is electronically synchronized with the imaging.

Such methods cannot control the volume of contrast agent in a blood vessel accurately enough to ensure a uniform concentration. As a result, the frame-to-frame brightness of the contrast agent can vary as different ultrasound images are taken of the blood vessel, making interpretation of the ultrasound images difficult.

### SUMMARY OF THE INVENTION

Described herein are systems and methods that integrate the injection of contrast agents with imaging catheters.

In an embodiment, an imaging catheter comprises a catheter sheath and an imager, e.g., ultrasound transducer, which may be mounted on the sheath or slidably received within the sheath. The imaging catheter further comprises a contrast lumen having one or more exit ports for injecting contrast agent into the patient. The contrast lumen extends along the catheter sheath and may be external to or integrated into the catheter sheath. Preferably, the exit port of the contrast lumen is positioned along the catheter sheath at a relatively short known distance, e.g., 20 cm or less, from the imager. The catheter may include multiple contrast lumens for injecting different types of contrast agents.

The imaging catheter with the integrated contrast agent injector advantageously reduces the volume of contrast agent that needs to be injected into the patient by injecting the contrast agent locally near the region to be imaged. The imaging catheter also provides easier synchronization between the injection of contrast agent and imaging. This is because the exit port of the contrast lumen is located at a short known distance from the imager, making it easier to estimate the time for the injected contrast agent to reach the region being imaged by the imager. In addition, the imaging catheter provides more precise control over the volume of contrast agent in a particular blood vessel by injecting the contrast agent directly into the blood vessel.

In an embodiment, a synchronizing controller is provided to automatically synchronize the injection of contrast agent with the imaging. The synchronizing controller controls the injection rate by controlling a pump, e.g., an electric pump, that pumps the contrast agent into the contrast lumen. In one embodiment, the synchronizing controller injects contrast agent at a uniform rate during imaging to provide a more consistent image brightness.

In another embodiment, drug-filled microbubbles in combination with ultrasound imaging are used to deliver a controlled drug dose to a specific treatment site. In this embodiment, the drug-filled microbubbles are delivered to the treatment site and subjected to high-level ultrasound energy to burst the microbubbles and release the drug into the treatment site. The amount of microbubbles that are ruptured, and hence the amount of the drug released into the treatment site, is determined by examining images taken before and after the microbubble bursting. This cycle of bursting microbubbles and determining the amount of the drug released can be repeated until a desired drug dose has been delivered to the treatment site.

Other systems, methods, features and advantages of the invention will be or will become apparent to one with skill in the art upon examination of the following figures and detailed description. It is intended that all such additional systems, methods, features and advantages be included within this description, be within the scope of the invention, and be protected by the accompanying claims.

### BRIEF DESCRIPTION OF THE FIGURES

In order to better appreciate how the above-recited and other advantages and objects of the present inventions are objected, a more particular description of the invention briefly described above will be rendered by reference to specific embodiments thereof, which are illustrated in the accompanying drawings. It should be noted that the components in the figures are not necessarily to scale, emphasis instead being placed upon illustrating the principles of the invention. Moreover, in the figures, like reference numerals designate corresponding parts throughout the different views. However, like parts do not always have like reference numerals. Moreover, all illustrations are intended to convey concepts, where relative sizes, shapes and other detailed attributes may be illustrated schematically rather than literally or precisely.
Fig. 1 shows an imaging catheter with an integrated contrast agent injector according to an embodiment of the present invention.
Fig. 2 shows an imaging catheter with an integrated contrast agent injector and a synchronizing controller for synchronizing contrast agent injection and imaging according to an embodiment of the present invention.
Fig. 3 shows an imaging catheter with an integrated contrast agent injector and a slidable imaging core according to an embodiment of the present invention.
Fig. 4 shows an imaging catheter with an integrated contrast agent injector and an inflatable/deflatable balloon according to an embodiment of the present invention.
Figs 5a-5c show an imaging catheter with a contrast agent lumen integrated in the catheter sheath according to an embodiment of the present invention.
Fig. 6 shows an example of delivering drug-filled microbubbles to a treatment site using a catheter with an integrated contrast agent injector according to an embodiment of the present invention.
Fig. 7 shows a flow diagram for a method of delivering drug-filled microbubbles to a treatment site according to an embodiment of the present invention.

### DETAILED DESCRIPTION

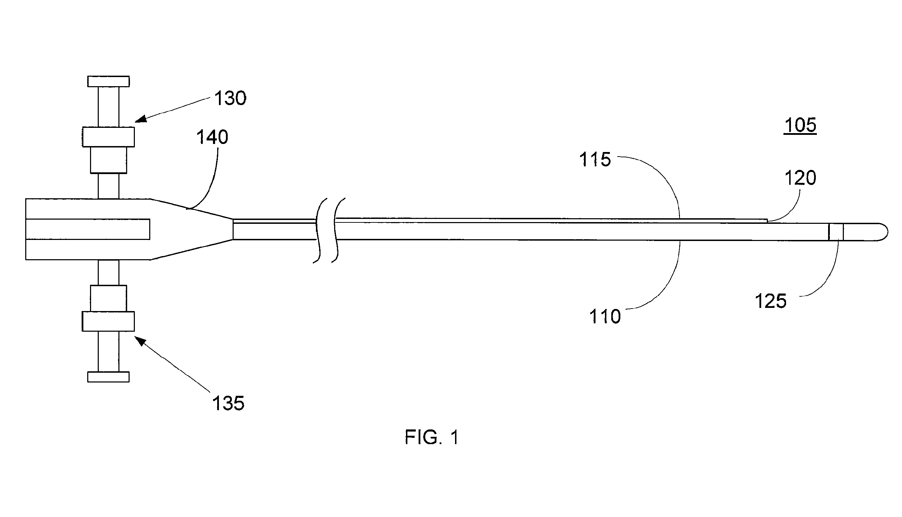

Fig. 1 shows an imaging catheter 105 apparatus with an integrated contrast agent injector according to an embodiment of the present invention. The catheter 105 comprises an elongated catheter sheath 110 and a contrast agent lumen 115 that extends along the catheter sheath 110. In a preferred embodiment, the catheter sheath 110 and contrast agent lumen 115 are adapted to be inserted into a patient's blood vessel, e.g., coronary artery, for contrast enhanced imaging within the blood vessel. The catheter may be adapted to be inserted in other passages in the body for imaging, e.g., the esophagus or urethra. The catheter sheath 110 and contrast lumen 115 may be made of a variety of polymeric materials, such as polytetrafluoroethylene (PTFE), polyethylene, PEEK, PEBAX or the like. The contrast lumen 115 includes an exit port 120 at its distal end for injecting contrast agent into the blood vessel.

The catheter 105 further comprises an ultrasound imager 125. The ultrasound imager 125 may comprise one or more ultrasound transducers, e.g., piezoelectric transducers or capacitive micromachined transducers (CMUTs). The ultrasound imager 125 may be mounted on the catheter sheath 110. Alternatively, the ultrasound imager 125 may be part of an imaging core that is slidably received within an inner lumen of the catheter sheath 110. The imager 125 may be side-looking and/or forward looking. Examples of intravascular catheters having side-looking and/or forward looking imagers can be found, for example, in U.S. Patent Application No. 11/104,865, filed on April 12, 2005, published as US 2006/0241478 A1.

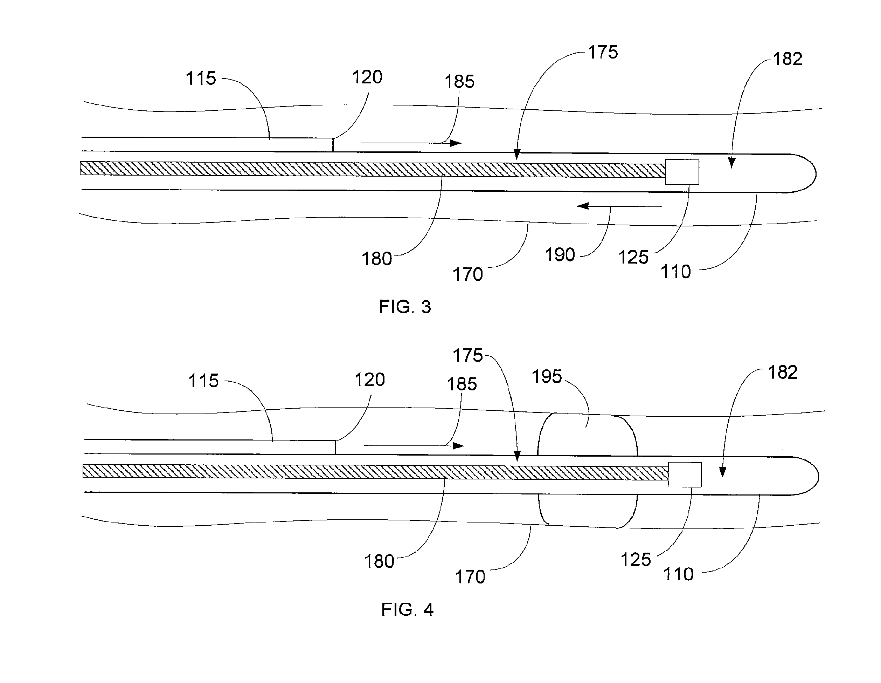

Fig. 3 shows a cross-sectional view of an exemplary imaging core 175 slidably received within the inner lumen 182 of the catheter sheath 110. The imaging core 175 comprises a drive cable 180 and an ultrasound imager 125 attached to the distal end of the drive cable 180. In this embodiment, the catheter sheath 110 may comprise a sonolucent material to allow ultrasound imaging through the sheath 110. The drive cable 180 may be used to rotate the ultrasound imager 125 within the catheter sheath 110 to mechanically scan a cross-sectional image of the blood vessel. The drive cable 180 may also be used to slide the ultrasound imager 125 within the catheter sheath. For example, the ultrasound imager 125 may be pulled backed within the catheter sheath 110 to image along a length of the blood vessel. The arrow 190 indicates the pullback direction.

Returning to Fig. 1, the catheter 105 further comprises a flush port 135 fluidly coupled to the inner lumen 182 of the catheter sheath 110 by a hub 140. The flush port 135 may be used, e.g., to inject saline into the inner lumen 182 of the catheter sheath 110 to enhance acoustic coupling between the ultrasound imager 125 and the catheter sheath 110. The catheter 105 also comprises a contrast agent injection port 130 fluidly coupled to the contrast lumen 115 for injecting contrast agent into the blood vessel via the contrast lumen 115. The contrast port 130 may use the same type of port typically used to flush saline into the catheter. Contrast agent may be injected into the contrast port 130 manually with a syringe, electronically with an electric pump, pressurized balder, or other means. The rate of injection of contrast agent into the blood vessel from the exit port 120 can be precisely controlled by controlling the rate of injection of contrast agent into the contrast port 130. Although the exit port 120 of the contrast lumen 115 is shown being located proximally from the imager 125, the exit port 120 may also be located distally from the imager 125.

The imaging catheter 105 according to the preferred embodiment provides several advantages over prior art methods in which contrast agent is injected into the patient at a injection point located away from the region of interest by an intravenous drip or a syringe. In these prior art methods, the patient's circulatory system not only carries injected contrast agent to the region of interest, but also distributes injected contrast agent throughout the vascular system. As a result, a larger quantity of contrast agent must be injected into the patient than needed to locally image the region of interest. The imaging catheter 105 reduces the quantity of contrast agent that needs to be injected into the patient by injecting the contrast agent locally near the region of interest from the exit port 120 of the catheter 105. For example, the catheter 105 allows the contrast agent to be injected into the same vascular structure, e.g., artery, as the region of interest.

The imaging catheter 105 also provides easier synchronization between the injection of contrast agent and imaging. This is because the exit port 120 of the catheter lumen 115 injects the contrast agent at a short known distance, e.g., 20 cm or less, from the imager 125, making it much easier to estimate the time for the contrast agent to reach the region of interest being imaged by the imager 125. The imager 125 may be aligned with the region of interest by using non-contrast imaging to locate the region of interest. Contrast agent can then be injected, e.g., to image fine structures within the region of interest. For a slidable imaging core 175, the distance between the exit port 120 can be determined using the known position of the exit port 120 along the sheath 110 and monitoring the relative position of the imaging core 125 within the catheter sheath 110.

The imaging catheter 105 also provides more precise control over the volume of contrast agent in a blood vessel by injecting contrast agent directly into the blood vessel. In prior art methods where the contrast agent is injected by an intravenous drip or a syringe, it is difficult to control the volume of contrast agent in a particular blood vessel because the injected contrast agent also flows to other blood vessels throughout the vascular system. An advantage of controlling the volume of contrast agent is that the volume of contrast agent in the blood flow can be controlled to ensure that a certain amount of blood continues to flow to downstream tissue (unlike the injection of x-ray contrast or a saline flush), thereby minimizing ischemic insult to downstream tissue.

The imaging catheter 105 may be used for contrast enhanced imaging of vulnerable plaque in the blood vessel, the diameter of the blood vessel, the vasa vasorum associated with the blood vessel, and the like. The imaging catheter 105 may also be used for contrast enhanced imaging of surrounding tissue by imaging the perfusion of contrast agent from the blood vessel into microvessels feeding blood to the surrounding tissue. The imaging catheter 105 may also be used to inject a targeted contrast agent into the blood vessel to image a certain tissue type, e.g., for tissue characterization. To do this, the targeted contrast agent may contain a ligand that is attracted to receptors of the tissue of interest. The imaging catheter 105 may also be used to inject a contrast agent containing a drug, or other pharmaceutical agent. For example, the contrast agent may comprise microbubbles containing the drug that burst and release the drug when subjected to sufficiently high ultrasound energy. Methods of delivering a drug in a patient using drug-filled microbubbles are discussed further below. The imaging catheter 105 may also be used to image the myocardium in the heart and other structures in the body.

The imaging catheter 105 may also be used to measure blood flow in a blood vessel. This may be done by measuring the transit time of contrast agent from the exit port 120 to the imager 125, and dividing the transit time by the known distance between the exit port 120 and the contrast lumen 115. A method for measuring blood flow according to an exemplary embodiment will now be discussed with reference to Fig. 3. In this embodiment, a bolus of contrast agent (not shown) is released into the blood vessel from the exit port 120 upstream of the imager 125. The arrow 185 indicates the direction of blood flow, which carries the bolus of contrast agent from the exit port 120 to the imager 125. The imager 125 is then activated to detect an increase in signal strength caused by the bolus passing the ultrasonic field of the imager 125, and therefore determine the transit time of the bolus from the exit port 120 to the imager 125. Determining the volume of flow through the vessel provides a means of accessing the heath of the vessel. Depending on the point at which flow is measured, this can be used to access cardiac output, fractional flow reserve, coronary flow reserve or other flow related physiologic purpose. More details of using contrast agents to measure blood flow can be found, for example, in PCT Application WO 2005/070299, titled "Methods and Apparatus for Medical Imaging," filed on January 14, 2005.

Contrast imaging can be used to calculate or observe other characteristics. For example, blood flow can be calculated by injecting a pulse of contrast agent into the bloodstream and using ultrasound imaging to measure the volume of microbubbles in a period of time to calculate blood flow. Contrast imaging can be used to calculate micro-vessel density in a vessel wall, blood velocity, and stenosis (narrowing of the blood vessel) by measuring blood velocity proximal to, inside of, and distal to the stenosis. Contrast imaging can also be used to determine lumen border by detection of contrast agent density.

Fig. 4 shows the imaging catheter 105 comprising an inflatable/deflatable balloon 195 according to an exemplary embodiment. In this embodiment, the balloon 195 is located between the exit port 120 and the imager 125, and is inflated by the injection of inflation fluid into the balloon 195 via an inflation lumen (not shown) within the catheter sheath 120. In one embodiment, the balloon 195 is used to create a high concentration of contrast agent in the region of interest. This is done by inflating the balloon 195 within the blood vessel 170, as shown in Fig. 4, and injecting contrast agent into the blood vessel from the exit port 120. The inflated balloon 195 temporarily blocks blood flow causing a high concentration of contrast agent to develop behind the balloon 195. The balloon 195 is then deflated to release the high concentration of contrast agent, which is carried by the blood flow to the region of interest within the imaging field of the imager 125. The balloon 195 may also be used to deploy a stent in the blood vessel, dissolve plaque in the blood vessel or other purpose.

Although Fig. 1 shows the imaging catheter 105 comprising one contrast lumen 115, the imaging catheter 105 may comprise multiple contrast lumens. For example, the imaging catheter 105 may comprise multiple contrast lumens to inject different types of contrast agents into the blood vessel. For example, one contrast lumen may be used to inject non-drug-filled microbubbles into the blood vessel to image the blood vessel and identify an area for treatment, e.g., atherosclerotic lesion. Another contrast lumen may then be used to inject drug-filled microbubbles into the blood vessel to deliver a drug to the treatment area. In these embodiments, the catheter apparatus may comprise multiple contrast injection ports 130, one for each contrast lumen.

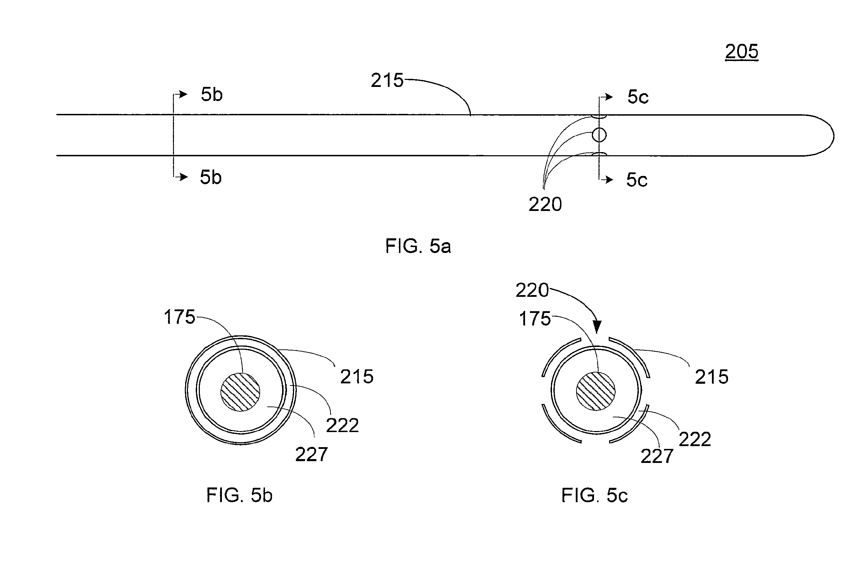

Further, the contrast lumen 115 may be external to the catheter sheath or integrated into the catheter sheath. Figs. 5a-5c show an exemplary embodiment of an imaging catheter 205, in which a contrast lumen 222 is integrated into the catheter sheath 215. In this exemplary embodiment, the contrast lumen 222 surrounds the working lumen 227 that receives the imaging core 175, as shown in Figs. 5b and 5c. In this exemplary embodiment, the catheter 205 comprises a plurality of exit ports 220 for the contrast lumen 222 arranged along the circumference of the catheter sheath 215. The annular arrangement of exit ports 220 provides more uniform injection of contrast agent around the circumference of the catheter sheath 215. Preferably, the contrast lumen 222 does not extend beyond the exit ports 220 in the catheter sheath 215. Different numbers and/or arrangements of exit ports may be used depending on the desired injection pattern. For example, the exit ports may be arranged along a spiral around the circumference of the catheter.

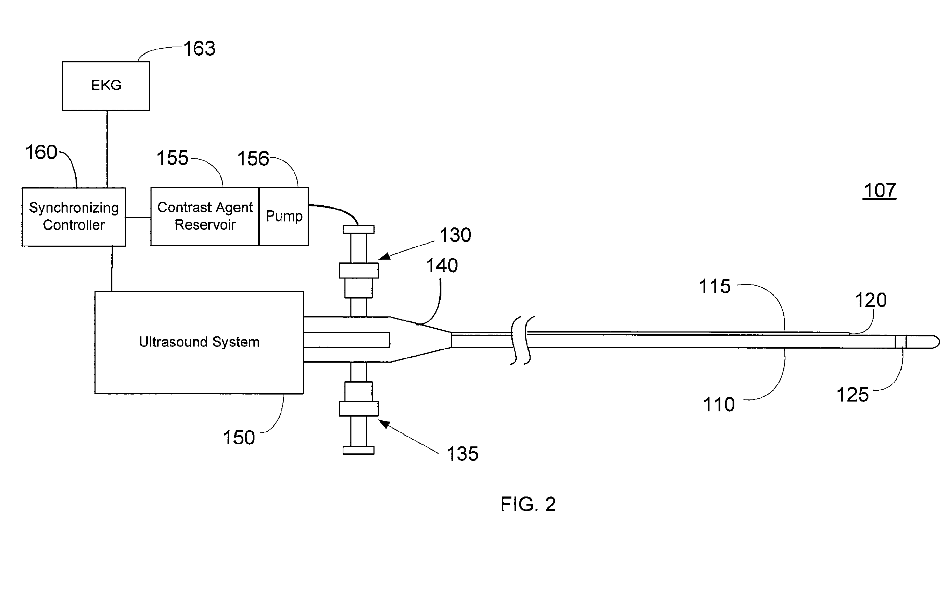

Fig. 2 shows an imaging catheter apparatus 107 for synchronizing the injection of contrast agent with imaging according to an exemplary embodiment. The catheter apparatus 107 comprises an ultrasound system 150 for driving the ultrasound imager 125 of the catheter 105, e.g., with transmit pulses, and for processing echo signals from the imager 125 into an ultrasound image. The imager 125 may be fixed to the end of the catheter 105 or mounted to the end of an imaging core 175 received with the catheter sheath 110. The imager 125 may comprise one transducer or a transducer array. For the imaging core embodiment, the ultrasound system may include a motor drive unit (MDU) for rotating and longitudinally translating the imaging core 175 within the catheter sheath 110. The catheter apparatus 107 also comprises a contrast agent reservoir 155 and pump 156 fluidly coupled to the contrast port 130. The pump 156 may comprise an electrical pump that pumps out contrast agent from the reservoir 155 based on an electrical signal. The catheter apparatus 107 further comprises a synchronizing controller 160 that electronically controls the ultrasound system 150 and the pump 156 to synchronize the injection of contrast agent with imaging. The synchronizing controller 160 may comprise a processor that executes instructions for performing the synchronization and may be integrated in the ultrasound system 150. In an exemplary embodiment, the synchronizing controller 160 controls the rate at which the pump 156 pumps out contrast agent from the reservoir 155, and thus the injection rate of the contrast agent. The synchronizing controller 160 may also control the acquisition of images from the imager 125. For the imaging core embodiment, the controller 160 may also control the rotational speed of the imaging core 175, and/or the pullback of the imaging core 175 within the catheter sheath 110.

A method for synchronizing the injection of contrast agent with imaging during a pullback procedure will now be described. In this embodiment, the synchronizing controller 160 activates the imager 125 and pulls back the imager 125, e.g., at a uniform rate, with the MDU of the ultrasound system 150. As the imager 125 is pulled back, the imager 125 may acquire cross-sectional images of the blood vessel, e.g., at evenly spaced intervals. The pull back procedure can last several minutes depending on the rate of pullback and the length of blood vessel being imaged. During the pullback procedure, the synchronizing controller 160 may control the pump 156 to inject contrast agent into the blood vessel at a uniform rate. The uniform injection of contrast agent during pullback provides a more uniform concentration of contrast agent in the blood. This results in more uniform contrast imaging along the entire length of the pullback. In this embodiment, the exit port 120 may be located far enough toward the proximal end of the catheter so that the exit port 120 remains proximal to the imager 125 throughout the pullback.

In one embodiment, the synchronizing controller 160 injects contrast agent into the blood vessel for a period of time before initiating pullback to allow the surrounding tissue time to absorb the contrast agent and the concentration of contrast agent in the surrounding tissue to reach a steady-state. The period of time may be based on a predetermined estimate or measurement of the absorption time for the surrounding tissue. Typically, the flow rate of blood in microvessels is typically 10 to 20 times slower than in the blood vessel. The period of time may also be determined in real time by analyzing images acquired by the imager 125 during the initial contrast injection. In this example, the absorption of contrast agent into the surrounding tissue may be determined based on areas of the surrounding tissue that appear bright in the ultrasound images due to the presence of the contrast agent. This analysis may be performed by the synchronizing controller 160, which may receive the ultrasound images from an image processor in the ultrasound system 150.

The catheter apparatus 205 is advantageous over prior art methods in which contrast agent is injected into the patient by an intravenous drip, or manual injection through a syringe or guide catheter. In these prior art methods, it is difficult to control the volume of contrast agent in the blood vessel accurately enough to provide a uniform concentration during pullback. As a result, the frame-to-frame brightness of the contrast agent is not uniform, making interpretation of the ultrasound images more difficult. The catheter apparatus 205 addresses this problem by providing a more uniform concentration of contrast agent in the blood during the entire pullback procedure.

In an exemplary embodiment, the synchronizing controller 160 is coupled to an electrocardiogram (EKG) monitor 163 to synchronize the acquisition of images from the imager 125 with the cardiac cycles of the patient. During each cardiac cycle, the blood vessel expands and contracts due to the pumping action of the heart. The resulting cardiac motion in images of the blood vessel can be reduced by acquiring the images at the same phase in the cardiac cycles. To do this, the EKG monitor 163 may send a signal to the synchronizing controller 160 indicating when the desired phase occurs in each cardiac cycle, and the synchronizing controller 160 may trigger an image acquisition and/or contrast agent injection when the signal indicates that the desired phase has occurred.

The synchronizing controller 160 may also control the injection rate based on the brightness of the contrast agent in the ultrasound images. For example, the synchronizing controller 160 may monitor the image brightness from the ultrasound image processor. When the image brightness decreases, the controller 160 may increase the injection rate of the contrast agent, and when the image brightness increases, the controller 160 may decrease the injection rate. Thus, the injection rate of the contrast agent can be adjusted based on image feedback to maintain a more uniform brightness in the ultrasound images.

The contrast agent injection may also be synchronized with a particular blood pressure. In this embodiment, the pressure may be measured, e.g., using a pressure wire. The contrast agent injection may also be synchronized with a particular volume of blood flow.

The contrast agent may be injected in an injection pulse, e.g., a square pulse (i.e., uniform injection over a period or time), a sloped pulse, or other shaped pulse. For an injection pulse, image acquisition may be triggered at the beginning of injection (or a fixed delay after) or at the end of the injection (or a fixed delay after). The image acquisition may also be triggered at the peak of an injection pulse (or a fixed delay after), e.g., for a nonuniform injection pulse. For a complex pulse shape, imaging can be triggered at any inflection point or at a fixed delay afterwards.

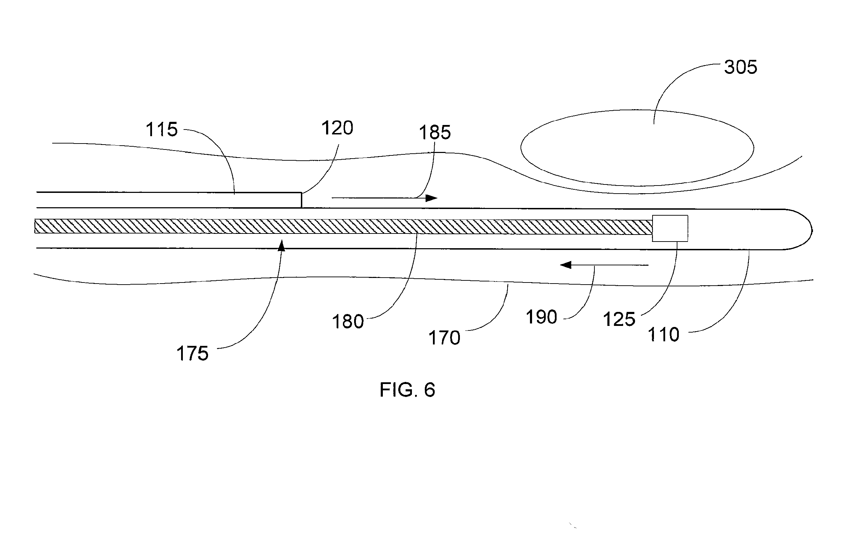

A method for locally delivering a controlled dose of a drug or other pharmaceutical agent to a treatment site using drug-filled microbubbles will now be described with reference to Fig. 6. Fig. 6 shows an example of the catheter in a blood vessel positioned at a treatment site 305. The treatment site 305 may be, e.g., an atherosclerotic lesion that can rupture and cause a blood clot if left untreated. In this embodiment, the drug-filled microbubbles are adapted to burst and release the drug when they are subjected to sufficiently high ultrasound energy and/or ultrasound energy within a certain frequency range. The drug-filled microbubbles within the treatment site may be imaged using low-level ultrasound energy that is insufficient to burst the microbubbles. The drug-filled microbubbles may be targeted microbubbles that target certain tissue types.

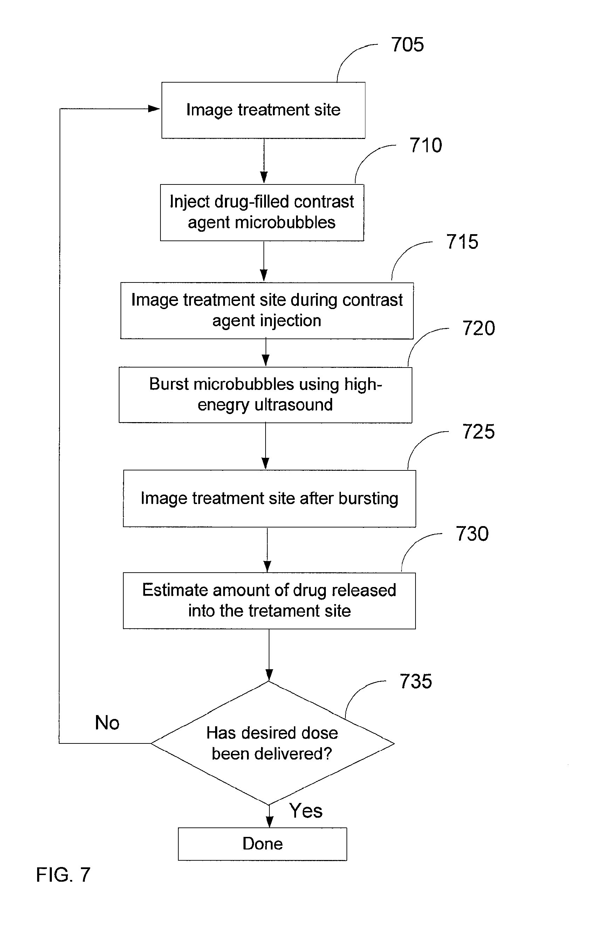

The treatment site 305 may be identified before the delivery of the drug by first imaging the blood vessel and analyzing the resulting images to identify the area to be treated. The blood vessel may be imaged, e.g., using non-drug-filled microbubbles or no contrast agent. After the treatment site has been identified, a controlled dose of the drug or pharmaceutical agent may be delivered to the treatment site 305 using the method described in the flowchart show in Fig. 7.

Turing now to the flowchart of Fig. 7, in step 705, the catheter is used to image the blood vessel including the treatment site 305. This may be done by pulling back the imager 125 and acquiring cross-sectional images at different positions along the blood vessel. The ultrasound console may then aggregate the images to construct a three-dimensional image of the blood vessel.

In step 710, microbubbles containing the drug or pharmaceutical agent is injected into the blood vessel near the treatment site 305. Preferably, the drug-filled microbubbles are released upstream of the treatment site 305. The drug-filled microbubbles perfuse into the treatment site 305, increasing the echocentricity of the treatment site 305.

In step 715, the catheter is used to image the treatment site 305 as the microbubbles perfuse into the treatment site 305. The perfusion of the microbubbles into the treatment site 305 causes the image brightness of the treatment site 305 to increase. The image brightness can be used to estimate the concentration of the unreleased drug in the treatment site 305. This is because the image brightness is a function of the concentration of microbubbles in the treatment site 305. The greater the image brightness, the higher the concentration of microbubbles, and hence the drug contained in the microbubbles. Preferably, the treatment site 305 is imaged using low-level ultrasound energy that is insufficient to burst the microbubbles. The image brightness of the treatment site 305 is monitored to determine when a desired concentration of the microbubbles containing the drug has been reached.

In step 720, when the desired concentration has been reached, the imager 125 is energized to an energy level sufficient to burst the microbubbles in the treatment site 305, thereby releasing the drug contained in the microbubbles into the treatment site 305. The imager 125 may be pulled back as the imager 125 is energized to burst the microbubbles along the entire length of the treatment site 305.

In step 725, the treatment site 305 is imaged after the imager 125 has been energized to determine the post-energizing microbubble concentration. Since additional microbubbles may perfuse into the treatment site 305 between the time the microbubbles burst and the time the post-energizing image is acquired, the post-energizing microbubble concentration may be adjusted to take this into account, e.g., based on the perfusion rate of microbubbles into the treatment site 305.

In step 730, the drug dose released in the treatment site 305 by the bursting of the microbubbles is estimated. This may be done by subtracting the post-energizing microbubble concentration from the pre-energizing microbubble concentration to determine the drug concentration released into the treatment site 305 and using the volume of the treatment site 305 to determine the drug dose. The volume of the treatment site 305 may be estimated based on a three-dimensional ultrasound image of the treatments site 305.

In step 735, the dose of the released drug is recorded and compared to the desired total dose to be delivered to the treatment site 305. If the desired total dose has not been reached, then steps 705 through 735 may be repeated until the desired amount of drug has been delivered to the treatment site 305.

Therefore, the drug delivery method enables a physician to more precisely deliver a controlled drug dose to a specific treatment site 305 in the body. Further, the drug delivery method reduces the amount of the drug that is delivered to other areas of the body outside of the treatments site 305. This is because the drug-filled microbubbles are injected locally near the treatment site 305 and controllably destroyed within the treatment site 305 to release the drug in the treatments site 305. The targeted delivery of the drug to the treatment site 305 is important, e.g., when the drug is harmful to surrounding healthy tissue.

The drug delivery method is based on the principle that the concentration of contrast agent microbubbles can be determined analytically by comparing the reflected ultrasound energy before and after administration of the contrast agent. By knowing the microbubble concentration, an estimation of the number of microbubbles and, therefore, the volume of the drug or pharmaceutical agent contained within the microbubbles can be calculated. Since the drug or pharmaceutical agent is inert until the microbubbles are destroyed, controlled ultrasound can be applied in short pulses to incrementally burst the microbubbles, and imaging before and after microbubble bursting can be used to determine the amount of drug released in the treatment site. This cycle can be continued until the proper dose of the drug or pharmaceutical agent has been delivered.

The drug-filled microbubbles may be injected into the patient using an imaging catheter with an integrated injector or a separate injection device. For example, the microbubbles may be injected using a separate guide catheter, or by an external means such an intravenous drip or a syringe. Also, the microbubbles may be imaged and destroyed using the same transducer or different transducers. Further, the drug-filled microbubbles may comprise microbubbles that have a frequency-generated non-linear response. This allows ultrasound waves reflected from the microbubbles to be isolated from ultrasound waves reflected from surrounding tissue, e.g. using a filter that filters out ultrasound waves at the fundamental frequency (i.e., frequency of the transmit signal). For example, the microbubbles may comprise harmonic microbubbles, subharmonic microbubbles, etc.

## Claims

1. A contrast imaging system, comprising:
an elongated catheter sheath (110; 215);
an imager (125) fixed to or received within the catheter sheath (110; 215), wherein the imager (125) acquires ultrasound images by emitting ultrasonic waves and receiving reflected ultrasonic waves;
a contrast lumen (115; 222) having a proximal end and a distal end, wherein the contrast lumen (115) extends along the catheter sheath (110; 215) and has an exit port (120; 220) at its distal end;
a pump (156) fluidly coupled to the proximal end of the contrast lumen (115; 222);
an ultrasound system (150) coupled to the imager (125) for driving and receiving signals from the imager (125); and
a controller (160) coupled to the pump and the ultrasound system for synchronizing injection of contrast agent from the exit port (120; 220) with the acquisition of images by the imager (125),
**characterized in that** the controller (160) is configured to control an injection rate of the contrast agent based on brightness of the contrast agent in the ultrasound images.

2. The system of claim 1, wherein the imager (125) comprises an ultrasound transducer.

3. The system of claim 2, wherein the exit port (120; 220) is located twenty centimeters or less from the transducer.

4. The system of claim 1, wherein the contrast lumen (115; 222) is integrated into the catheter sheath.

5. The system of claim 1, further comprising an imaging core (175) slidably received within the catheter sheath (110; 215) wherein the imager (125) is mounted to a distal end of the imaging core (175).

6. The system of claim 1, wherein the pump (156) comprises an electrical pump.

7. The system of claim 1, wherein the contrast lumen (115; 222) has a plurality of exit ports (220) at its distal end.

8. The system of claim 1, further comprising a second contrast lumen that is separate from the first contrast lumen (115; 222).

9. The system of claim 1, wherein the controller (160) is configured to inject contrast agent at a substantially constant rate using the pump (156) while the imager acquires ultrasound images.

10. The system of claim 1, wherein the catheter sheath (110; 215) is adopted to be inserted into an artery.

11. The system of claim 5, wherein the controller (160) is configured to inject contrast agent at a substantially constant rate using the pump (156) while the imaging core (175) is pulled back to acquire ultrasound images.

12. The contrast imaging system of claim 1, further comprising an injection port fluidly coupled to the proximal end of the contrast lumen (115; 222) for injecting contrast agent into the contrast lumen (115; 222).

## Patentansprüche

1. Kontrastabbildungssystem, das aufweist:
einen länglichen Kathetermantel (110; 215);
einen Bildwandler (125), der am Kathetermantel (110; 215) befestigt oder darin aufgenommen ist, wobei der Bildwandler (125) Ultraschallbilder durch Abstrahlen von Ultraschallwellen und Empfangen reflektierter Ultraschallwellen erstellt;
ein Kontrastlumen (115; 222) mit einem proximalen Ende und einem distalen Ende, wobei sich das Kontrastlumen (115) entlang des Kathetermantels (110; 215) erstreckt und eine Austrittsöffnung (120; 220) an seinem distalen Ende hat;
eine Pumpe (156), die mit dem proximalen Ende des Kontrastlumens (115; 222) fluidgekoppelt ist;
ein Ultraschallsystem (150), das mit dem Bildwandler (125) gekoppelt ist, zum Ansteuern und Empfangen von Signalen vom Bildwandler (125); und
eine Steuerung (160), die mit der Pumpe und dem Ultraschallsystem gekoppelt ist, zum Synchronisieren von Kontrastmittelinjektion aus der Austrittsöffnung (120; 220) mit der Erstellung von Bildern durch den Bildwandler (125),
**dadurch gekennzeichnet, dass** die Steuerung (160) so konfiguriert ist, dass sie eine Injektionsgeschwindigkeit des Kontrastmittels auf der Grundlage der Helligkeit des Kontrastmittels in den Ultraschallbildern steuert.

2. System nach Anspruch 1, wobei der Bildwandler (125) einen Ultraschallwandler aufweist.

3. System nach Anspruch 2, wobei die Austrittsöffnung (120; 220) höchstens zwanzig Zentimeter vom Wandler liegt.

4. System nach Anspruch 1, wobei das Kontrastlumen (115; 222) in den Kathetermantel integriert ist.

5. System nach Anspruch 1, ferner mit einem Abbildungskern (175), der im Kathetermantel (110; 215) verschiebbar aufgenommen ist, wobei der Bildwandler (125) an einem distalen Ende des Abbildungskerns (175) angebaut ist.

6. System nach Anspruch 1, wobei die Pumpe (156) eine elektrische Pumpe aufweist.

7. System nach Anspruch 1, wobei das Kontrastlumen (115; 222) mehrere Austrittsöffnungen (220) an seinem distalen Ende hat.

8. System nach Anspruch 1, ferner mit einem zweiten Kontrastlumen, das vom ersten Kontrastlumen (115; 222) getrennt ist.

9. System nach Anspruch 1, wobei die Steuerung (160) so konfiguriert ist, dass sie Kontrastmittel mit einer im Wesentlichen konstanten Geschwindigkeit mit Hilfe der Pumpe (156) injiziert, während der Bildwandler Ultraschallbilder erstellt.

10. System nach Anspruch 1, wobei der Kathetermantel (110; 215) geeignet ist, in eine Arterie eingeführt zu werden.

11. System nach Anspruch 5, wobei die Steuerung (160) so konfiguriert ist, dass sie Kontrastmittel mit einer im Wesentlichen konstanten Geschwindigkeit mit Hilfe der Pumpe (156) injiziert, während der Abbildungskern (175) zurückgezogen wird, um Ultraschallbilder zu erfassen.

12. Kontrastabbildungssystem nach Anspruch 1, ferner mit einer Injektionsöffnung, die mit dem proximalen Ende des Kontrastlumens (115; 222) fluidgekoppelt ist, zum Injizieren von Kontrastmittel in das Kontrastlumen (115; 222).

## Revendications

1. Système d'imagerie de contraste comprenant :
une gaine de cathéter allongée (110 ; 215) ;
un dispositif d'imagerie (125) fixé à ou reçu dans la gaine de cathéter (110 ; 215), où le dispositif d'imagerie (125) acquiert des images ultrasonores en émettant des ondes ultrasonores et en recevant les ondes ultrasonores réfléchies ;
une lumière de contraste (115 ; 222) ayant une extrémité proximale et une extrémité distale, où la lumière de contraste (115) s'étend le long de la gaine de cathéter (110 ; 215) et a un orifice de sortie (120 ; 220) à son extrémité distale ;
une pompe (156) couplée par fluide à l'extrémité proximale de la lumière de contraste (115 ; 222) ;
un système ultrasonore (150) couplé au dispositif d'imagerie (125) pour conduire et recevoir des signaux depuis le dispositif d'imagerie (125) ; et
un dispositif de commande (160) couplé à la pompe et au système ultrasonore pour synchroniser l'injection d'agent de contraste depuis l'orifice de sortie (120 ; 220) avec l'acquisition d'images par le dispositif d'imagerie (125),
**caractérisé en ce que** le dispositif de commande (160) est configuré pour commander une vitesse d'injection de l'agent de contraste sur la base de la brillance de l'agent de contraste dans les images ultra-sonores.

2. Système selon la revendication 1, où le dispositif d'imagerie (125) comprend un transducteur ultrasonore.

3. Système selon la revendication 2, où l'orifice de sortie (120 ; 220) est situé à 20 centimètres ou moins du transducteur.

4. Système selon la revendication 1, où la lumière de contraste (115 ; 222) est intégrée dans la gaine de cathéter.

5. Système selon la revendication 1, comprenant en outre une âme d'imagerie (175) reçue à coulissement dans la gaine de cathéter (110 ; 215) où le dispositif d'imagerie (125) est monté à une extrémité distale de l'âme d'imagerie (175).

6. Système selon la revendication 1, où la pompe (156) comprend une pompe électrique.

7. Système selon la revendication 1, où la lumière de contraste (115 ; 222) a une pluralité d'orifices de sortie (220) à son extrémité distale.

8. Système selon la revendication 1, comprenant en outre une seconde lumière de contraste qui est séparée de la première lumière de contraste (115 ; 222).

9. Système selon la revendication 1, où le dispositif de commande (160) est configuré pour injecter un agent de contraste à une vitesse sensiblement constante au moyen de la pompe (156) tandis que le dispositif d'imagerie acquiert des images ultrasonores.

10. Système selon la revendication 1, où la gaine de cathéter (110 ; 215) est adoptée pour être insérée dans une artère.

11. Système selon la revendication 5, où le dispositif de commande (160) est configuré pour injecter un agent de contraste à une vitesse sensiblement constante au moyen de la pompe (156) tandis que l'âme d'imagerie (175) est tirée en arrière pour acquérir des images ultrasonores.

12. Système d'imagerie de contraste selon la revendication 1, comprenant en outre un orifice d'injection couplé par fluide à l'extrémité proximale de la lumière du contraste (115 ; 222) pour injecter un agent de contraste dans la lumière de contraste (115 ; 222).
